# EUROPEAN PATENT APPLICATION

(11) **EP 3 327 717 A1**
(43) Date of publication of application: **30.05.2018**
(21) Application number: 16827695.4
(22) Date of filing: 14.07.2016
(51) Int. Cl.: G09G 5/00, H04N 5/64

(54) **INFORMATION PROCESSING DEVICE, METHOD FOR EVALUATING FATIGUE LEVEL, AND PROGRAM**

(30) Priority: 21.07.2015 JP 2015143616
(71) Applicant: Sony Interactive Entertainment Inc., Tokyo 108-0075 (JP)
(72) Inventor: HOSHINO, Masanori, Tokyo 108-0075 (JP)
(74) Representative: Müller Hoffmann & Partner
(86) International application number: PCT/JP2016/070786
(87) International publication number: WO 2017/014135

(57) **Abstract**

An information processing apparatus is provided which can properly evaluate a degree of fatigue of a user using a head-mounted display. The information processing apparatus includes: a state determining unit (51) that determines whether the head-mounted display is in a mounted state; a fatigue degree evaluating unit (53) that evaluates the degree of fatigue of the user wearing the head-mounted display on the basis of a cumulative wearing time obtained by accumulating times in which it is determined that the head-mounted display is in the mounted state; and a notifying unit (54) that provides the user with a notification corresponding to the degree of fatigue of the user.

## Description

### [Technical Field]

The present invention relates to an information processing apparatus, a fatigue degree evaluating method, and a program.

### [Background Art]

Head-mounted displays used in a state of being mounted on a head, as described in PTL 1 to 3, can provide a game space or the like in which a sense of immersion is enhanced. On the other hand, because of the enhanced sense of immersion, the user may be absorbed in the game without being aware of fatigue of the user himself/herself.

### [Citation List]

### [Patent Literature]

[PTL 1]
   JP 2004-205711A
[PTL 2]
   JP 2012-2889A
[PTL 3]
   JP 2013-210588A

### [Summary]

### [Technical Problems]

Accordingly, for example, when it is determined that the degree of fatigue of the user is high, the user can be made aware of fatigue of the user himself/herself by making a notification prompting the user to take a break. In order to realize the above-described notification, the degree of fatigue of the user which fatigue is caused by the use of a head-mounted display needs to be evaluated properly.

The degree of fatigue of the user may be evaluated according to a usage time of the head-mounted display, for example. However, supposing that a power-on time of the head-mounted display is used as the usage time of the head-mounted display, the usage time is accumulated when power remains on even in a case where the head-mounted display is detached and a break is taken. Therefore, the degree of fatigue of the user cannot be evaluated properly.

The present invention has been made in view of the above problems. It is an object of the present invention to provide an information processing apparatus, a fatigue degree evaluating method, and a program that can properly evaluate the degree of fatigue of a user using a head-mounted display.

### [Solution to Problems]

In order to solve the above problems, according to the present invention, there is provided an information processing apparatus including: a state determining unit configured to determine whether a head-mounted display is in a mounted state; a fatigue degree evaluating unit configured to evaluate a degree of fatigue of a user wearing the head-mounted display on a basis of a cumulative wearing time obtained by accumulating times in which it is determined that the head-mounted display is in the mounted state; and a notifying unit configured to provide the user with a notification corresponding to the degree of fatigue of the user.

In addition, in the information processing apparatus, the notifying unit may provide the notification when the cumulative wearing time exceeds a predetermined threshold value.

In addition, in the information processing apparatus, the state determining unit is able to further determine whether the head-mounted display is in a detached state, and the cumulative wearing time may be reset when a cumulative detachment time obtained by accumulating times in which it is determined that the head-mounted display is in the detached state exceeds a predetermined threshold value.

In addition, in the information processing apparatus, the state determining unit is able to further determine whether the head-mounted display is in a detached state, and subtraction from the cumulative wearing time may be performed according to a time in which it is determined that the head-mounted display is in the detached state.

In addition, in the information processing apparatus, the state determining unit is able to further determine whether the head-mounted display is in a detached state, and the cumulative wearing time may be reset when it is determined that the head-mounted display is in the detached state.

In addition, the information processing apparatus may further include a user identifying unit configured to identify the user wearing the head-mounted display, in which the fatigue degree evaluating unit may evaluate the degree of fatigue of the user on the basis of the cumulative wearing time obtained by accumulating times in which it is determined that the identified user wears the head-mounted display, and the notifying unit may provide the notification to the user when the cumulative wearing time of the identified user exceeds a predetermined threshold value.

In addition, according to the present invention, there is provided a fatigue degree evaluating method including: a step of determining whether a head-mounted display is in a mounted state; and a step of evaluating a degree of fatigue on a basis of a cumulative wearing time obtained by accumulating times in which it is determined that the head-mounted display is in the mounted state.

In addition, according to the present invention, there is provided a program for making a computer function as: state determining means for determining whether a head-mounted display is in a mounted state; fatigue degree evaluating means for evaluating a degree of fatigue of a user wearing the head-mounted display on a basis of a cumulative wearing time obtained by accumulating times in which it is determined that the head-mounted display is in the mounted state; and notifying means for providing the user with a notification corresponding to the degree of fatigue of the user. This program may be stored on a computer readable information storage medium.

### [Brief Description of Drawings]

[FIG. 1]

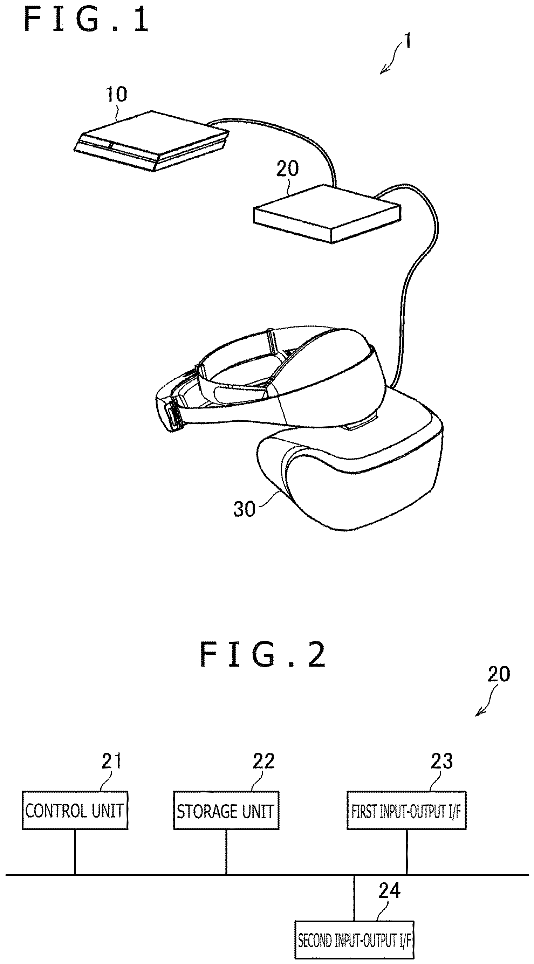

   FIG. 1 is a diagram illustrating an example of a general configuration of an information processing system according to a present embodiment.
[FIG. 2]
   FIG. 2 is a diagram illustrating an example of a hardware configuration of a relay apparatus according to the present embodiment.
[FIG. 3]

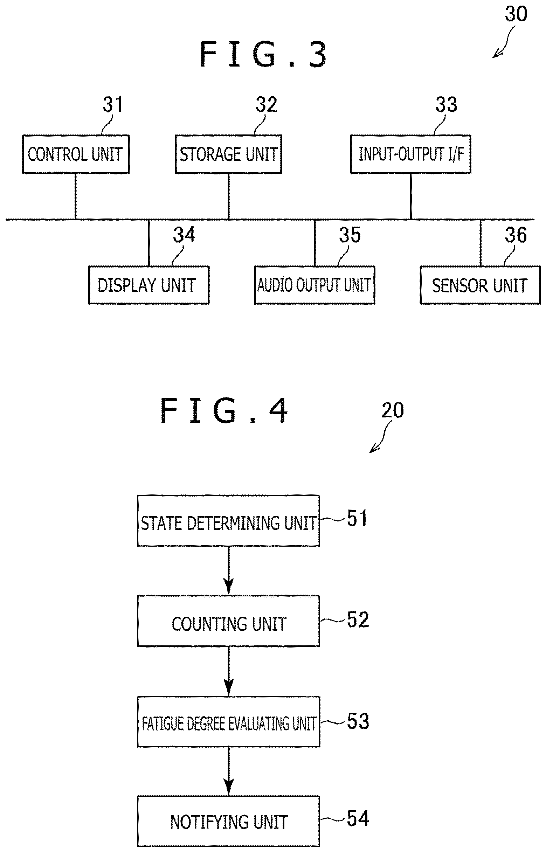

   FIG. 3 is a diagram illustrating an example of a hardware configuration of a head-mounted display (HMD) according to the present embodiment.
[FIG. 4]
   FIG. 4 is a functional block diagram illustrating an example of main functions implemented by the relay apparatus according to the present embodiment.
[FIG. 5A]

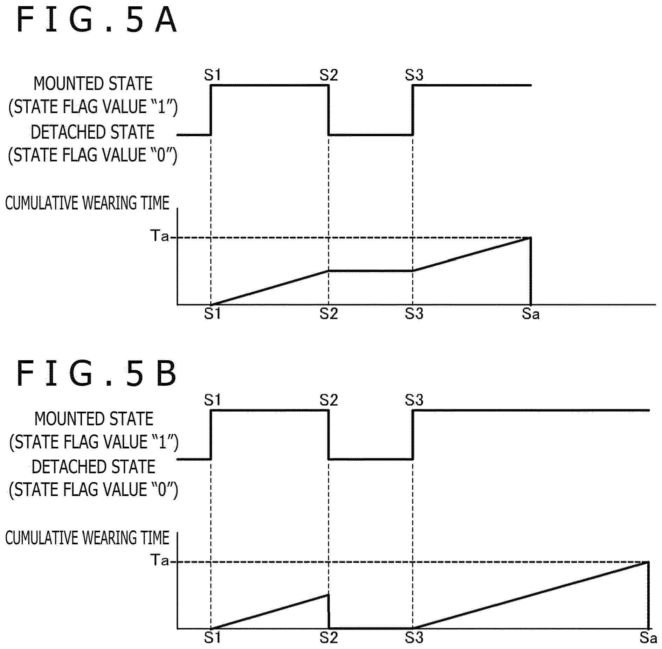

   FIG. 5A is a diagram illustrating an example of a cumulative wearing time graph corresponding to a mounted state and a detached state according to the embodiment.
[FIG. 5B]
   FIG. 5B is a diagram illustrating an example of a cumulative wearing time graph corresponding to the mounted state and the detached state according to the embodiment.
[FIG. 5C]

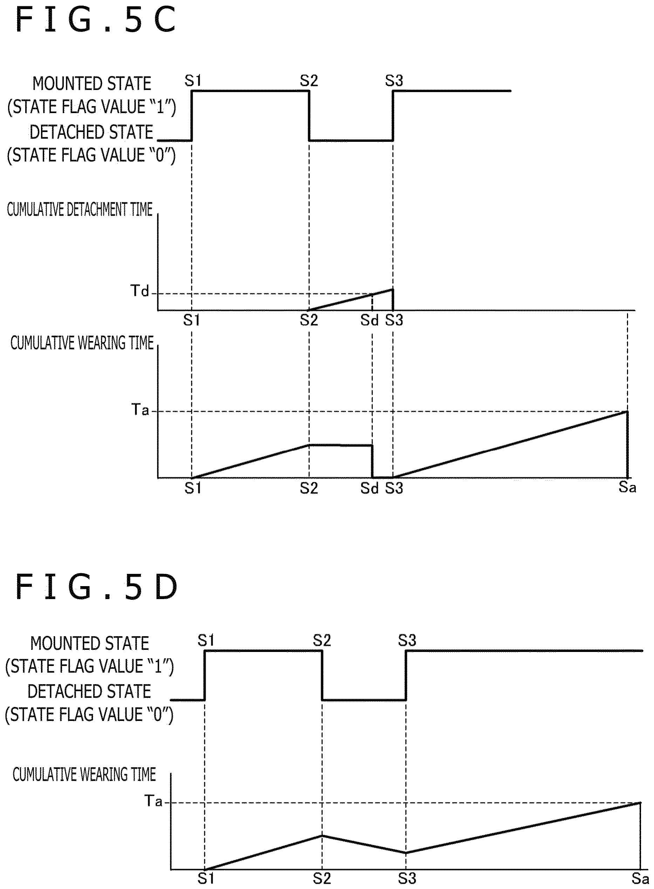

   FIG. 5C is a diagram illustrating an example of a cumulative wearing time graph corresponding to the mounted state and the detached state according to the embodiment.
[FIG. 5D]
   FIG. 5D is a diagram illustrating an example of a cumulative wearing time graph corresponding to the mounted state and the detached state according to the embodiment.
[FIG. 6]

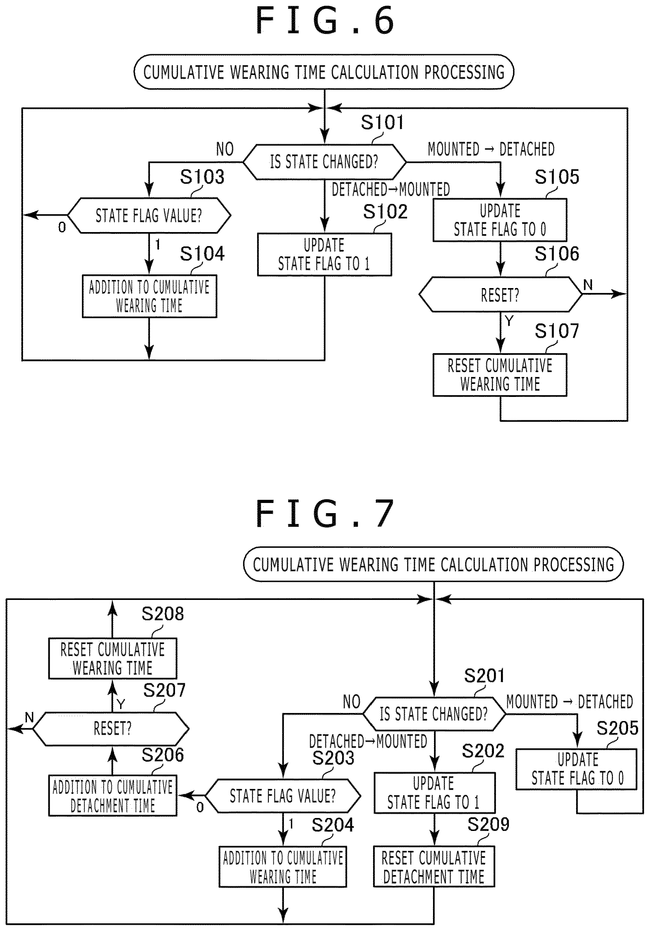

   FIG. 6 is a flowchart illustrating an example of cumulative wearing time calculation processing performed by the relay apparatus according to the present embodiment.
[FIG. 7]
   FIG. 7 is a flowchart illustrating another example of the cumulative wearing time calculation processing performed by the relay apparatus according to the present embodiment.

### [Description of Embodiment]

An embodiment of the present invention will hereinafter be described with reference to the drawings.

### System Configuration

FIG. 1 is a diagram illustrating an example of a general configuration of an information processing system 1 according to the present embodiment. As illustrated in FIG. 1, the information processing system 1 according to the present embodiment includes an entertainment apparatus 10, a relay apparatus 20, and an HMD 30. The entertainment apparatus 10 and the relay apparatus 20 are connected to each other, for example, via a high-definition multimedia interface (HDMI) (registered trademark) cable, a universal serial bus (USB) cable, or the like. The relay apparatus 20 and the HMD 30 are connected to each other, for example, via an HDMI cable, a USB cable, or the like.

The entertainment apparatus 10 is an information processing apparatus such, for example, as a game machine for home use, a digital versatile disc (DVD) player, a Blue-ray (registered trademark) player, or the like. The entertainment apparatus 10 according to the present embodiment outputs, to the relay apparatus 20, a video signal generated by, for example, executing an installed game program or reproducing an optical disk to be read as well as an audio signal representing sound.

The relay apparatus 20 is an information processing apparatus that relays between the entertainment apparatus 10 and the HMD 30. The relay apparatus 20 according to the present embodiment relays the video signal and the audio signal output from the entertainment apparatus 10 and thus outputs the video signal and the audio signal to the HMD 30.

FIG. 2 is a diagram illustrating an example of a hardware configuration of the relay apparatus 20 according to the present embodiment. As illustrated in FIG. 2, the relay apparatus 20 according to the present embodiment includes a control unit 21, a storage unit 22, a first input-output interface (I/F) 23, and a second input-output I/F 24.

The control unit 21 includes a program control device such as a central processing unit (CPU) or the like. The control unit 21 performs various kinds of information processing according to a program stored in the storage unit 22.

The storage unit 22 includes memory elements such as a random access memory (RAM) and a read only memory (ROM) or the like. The storage unit 22 stores the program executed by the control unit 21 and the like. The storage unit 22 also functions as a work memory of the control unit 21.

The first input-output I/F 23 is an interface for establishing connection between the relay apparatus 20 and the HMD 30, the interface being, for example, an HDMI port, a USB port, or the like. The first input-output I/F 23 outputs various kinds of signals to the HMD 30 according to instructions from the control unit 21. In addition, the first input-output I/F 23 receives a signal output from the HMD 30 and outputs the signal to the control unit 21.

The second input-output I/F 24 is an interface for establishing connection between the relay apparatus 20 and the entertainment apparatus 10, the interface being, for example, an HDMI port, a USB port, or the like. The second input-output I/F 24 outputs various kinds of signals to the entertainment apparatus 10 according to instructions from the control unit 21. In addition, the second input-output I/F 24 receives a signal output from the entertainment apparatus 10 and outputs the signal to the control unit 21.

The HMD 30 is an eye front mounting type display apparatus mounted on the head of a user and presenting two-dimensional or three-dimensional video or the like to the user by using a display device included within the HMD 30.

FIG. 3 is a diagram illustrating an example of a hardware configuration of the HMD 30 according to the present embodiment. As illustrated in FIG. 3, the HMD 30 according to the present embodiment includes a control unit 31, a storage unit 32, an input-output I/F 33, a display unit 34, an audio output unit 35, and a sensor unit 36.

The control unit 31 includes a program control device such as a CPU or the like. The control unit 31 performs various kinds of information processing according to a program stored in the storage unit 32.

The storage unit 32 includes memory elements such as a RAM and a ROM or the like. The storage unit 32 stores a program executed by the control unit 31 and the like. The storage unit 32 also functions as a work memory of the control unit 31.

The input-output I/F 33 is an interface for establishing connection between the relay apparatus 20 and the HMD 30, the interface being, for example, an HDMI port, a USB port, or the like. The input-output I/F 33 outputs various kinds of signals to the relay apparatus 20 according to instructions from the control unit 31. In addition, the input-output I/F 33 receives a signal output from the relay apparatus 20 and outputs the signal to the control unit 31.

The display unit 34 is a display device such, for example, as a liquid crystal display, an organic electroluminescence (EL) display, or the like. The display unit 34 displays video represented by the video signal output from the relay apparatus 20.

The audio output unit 35 is, for example, a speaker or the like. The audio output unit 35 outputs the sound represented by the audio signal output from the relay apparatus 20.

The sensor unit 36 is a sensor such, for example, as an infrared sensor, a contact sensor, or the like. The sensor unit 36 detects whether or not a user has put on the HMD 30. For example, whether or not the face of the user is located at a position facing the display unit 34 is detected using an infrared sensor, which measures a distance to an object by detecting reflected light obtained by irradiating the object with infrared light. Whether or not the user has put on the HMD 30 is thereby detected. Incidentally, a device capable of detecting the face of the user, such as a charge-coupled device (CCD) image sensor or another imaging element, may be included in place of the infrared sensor. In addition, when a contact sensor is provided at a position at which the HMD 30 is in contact with the user in a state in which the HMD 30 is mounted on the user, whether or not the user has put on the HMD 30 can be detected on the basis of whether or not the contact sensor has detected contact.

Then, the information processing system 1 according to the present embodiment is configured to evaluate a degree of fatigue of the user properly on the basis of a cumulative wearing time obtained by accumulating times in which the HMD 30 is determined to be mounted, under an idea that the degree of fatigue of the user increases according to an increase in time that the user wears the HMD 30. Functions implemented by the information processing system 1 according to the present embodiment will be described in detail in the following.

### Functional Block Diagram

FIG. 4 is a functional block diagram illustrating an example of main functions implemented by the relay apparatus 20 according to the present embodiment. As illustrated in FIG. 4, the relay apparatus 20 according to the present embodiment functionally includes, for example, a state determining unit 51, a counting unit 52, a fatigue degree evaluating unit 53, and a notifying unit 54. These functions are implemented by the control unit 21 by executing a program stored in the storage unit 22. This program may be provided in a state of being stored on various kinds of computer readable information storage media such, for example, as an optical disk and the like, or may be provided to the relay apparatus 20 via a communication network such as the Internet or the like.

The state determining unit 51 obtains a result of detection by the sensor unit 36 of the HMD 30, and determines the state of the HMD 30 according to the detection result at a predetermined frame rate (for example, at intervals of 1/60 of a second). The state of the HMD 30 which state is determined by the state determining unit 51 in the present embodiment indicates one of states at a time of a change from a state in which the HMD 30 is detached from the user (which state will hereinafter be referred to as a detached state) to a state in which the HMD 30 is mounted on the user (which state will hereinafter be referred to as a mounted state), at a time of a change from the mounted state to the detached state, and at a time of the occurrence of no change in the state of the HMD 30. In addition, the time of the occurrence of no change in the state of the HMD 30 indicates that the mounted state is maintained or that the detached state is maintained. Specifically, the state determining unit 51 determines that a change is made from the detached state to the mounted state at a point in time that the sensor unit 36 detects the user from a state of not detecting the user, and determines that a change is made from the mounted state to the detached state at a point in time that the sensor unit 36 does not detect the user from a state of detecting the user. Further, the state determining unit 51 determines that the state of the HMD 30 is not changed when the sensor unit 36 does not detect the user again from a state of not detecting the user or when the sensor unit 36 detects the user again from a state of detecting the user. Incidentally, the state determining unit 51 may determine that the HMD 30 is in the mounted state from a time point that the sensor unit 36 detects the user in a state of not detecting the user to a time point that the sensor unit 36 does not detect the user again, and determine that the HMD 30 is in the detached state from a time point that the sensor unit 36 does not detect the user in a state of detecting the user to a time point that the sensor unit 36 detects the user again.

In addition, the state determining unit 51 may determine the state of the HMD 30 on the basis of a moving image imaged by an external camera. For example, suppose that the external camera connected to the entertainment apparatus 10 is installed in such a position as to be able to image the user, and the state determining unit 51 may obtain a moving image of the user photographed by the external camera via the entertainment apparatus 10 at intervals of a predetermined time. The state determining unit 51 may then determine the mounted state or the detached state by image analysis of the obtained moving image. Incidentally, the state determining unit 51 may obtain a result of image analysis performed by the entertainment apparatus 10, and determine the state of the HMD 30 according to the result.

In addition, suppose in the present embodiment that whether the HMD 30 is in the mounted state or the detached state is managed by a state flag. Then, suppose in the present embodiment that the state determining unit 51 retains the state flag. In the present embodiment, the value of the state flag is "1" when the HMD 30 is in the mounted state, and the value of the state flag is "0" when the HMD 30 is in the detached state.

The counting unit 52 calculates a cumulative wearing time by accumulating times in which it is determined that the HMD 30 is in the mounted state. Then, suppose in the present embodiment that the counting unit 52 retains cumulative wearing time data indicating the calculated cumulative wearing time. Specifically, suppose that the counting unit 52 adds to the cumulative wearing time in a period in which it is determined that the HMD 30 is in the mounted state, and does not add to the cumulative wearing time in a period in which it is determined that the HMD 30 is in the detached state. In addition, suppose that the counting unit 52 resets the cumulative wearing time in predetermined timing. For example, the counting unit 52 resets the cumulative wearing time when the cumulative wearing time exceeds a predetermined threshold value (Ta).

FIG. 5A is a diagram illustrating an example of a cumulative wearing time graph corresponding to the mounted state and the detached state according to the present embodiment. FIG. 5A illustrates that the HMD 30 is changed from the detached state (state flag "0") to the mounted state (state flag "1") in timing S1, illustrates that the HMD 30 is in the mounted state in a period from timing S1 to timing S2, illustrates that the HMD 30 is changed from the mounted state to the detached state in timing S2, illustrates that the HMD 30 is in the detached state in a period from timing S2 to timing S3, illustrates that the HMD 30 is changed from the detached state to the mounted state in timing S3, and illustrates that the HMD 30 is in the mounted state after timing S3. As illustrated in FIG. 5A, first, addition to the cumulative wearing time is performed in a period in which it is determined that the HMD 30 is in the mounted state (timing S1 to timing S2) and after timing S3, and no addition to the cumulative wearing time is performed in a period in which it is determined that the HMD 30 is in the detached state (timing S2 to timing S3). That is, during the period during which it is determined that the HMD 30 is in the detached state (timing S2 to timing S3), the cumulative wearing time is maintained at a value at the time point of timing S2. Then, the cumulative wearing time is reset in timing Sa in which the cumulative wearing time exceeds a predetermined threshold value (Ta).

Further, the counting unit 52 may reset the cumulative wearing time when it is determined that the HMD 30 is changed from the mounted state to the detached state.

FIG. 5B is a diagram illustrating an example of a cumulative wearing time graph corresponding to the mounted state and the detached state according to the present embodiment. FIG. 5B illustrates that the HMD 30 is changed from the detached state to the mounted state in timing S1, illustrates that the HMD 30 is in the mounted state in a period from timing S1 to timing S2, illustrates that the HMD 30 is changed from the mounted state to the detached state in timing S2, illustrates that the HMD 30 is in the detached state in a period from timing S2 to timing S3, illustrates that the HMD 30 is changed from the detached state to the mounted state in timing S3, and illustrates that the HMD 30 is in the mounted state after timing S3. As illustrated in FIG. 5B, first, addition to the cumulative wearing time is performed in a period in which it is determined that the HMD 30 is in the mounted state (timing S1 to timing S2). Next, the cumulative wearing time is reset in timing S2 in which the HMD 30 is changed from the mounted state to the detached state. Then, no addition to the cumulative wearing time is performed in a period in which it is determined that the HMD 30 is in the detached state (timing S2 to timing S3). That is, during the period during which it is determined that the HMD 30 is in the detached state (timing S2 to timing S3), the cumulative wearing time is maintained in the reset state. Then, addition to the reset cumulative wearing time is performed again after timing S3 in which the HMD 30 is changed from the detached state to the mounted state. Incidentally, suppose that in FIG. 5B, the cumulative wearing time is reset also in timing Sa in which the cumulative wearing time exceeds a predetermined threshold value (Ta).

In addition, the counting unit 52 may further calculate a cumulative detachment time obtained by accumulating periods in which it is determined that the HMD 30 is in the detached state. Then, the counting unit 52 may reset the cumulative wearing time when the cumulative detachment time exceeds a predetermined threshold value (Td). In addition, suppose in this case that the counting unit 52 retains cumulative detachment time data indicating the calculated cumulative detachment time. In this case, because it is considered that the degree of fatigue of the user decreases according to the cumulative detachment time, or in other words, a break time during which the HMD 30 is not used, the cumulative wearing time is reset when the cumulative detachment time exceeds a predetermined threshold value considered to represent a sufficient break time.

FIG. 5C is a diagram illustrating an example of a cumulative wearing time graph corresponding to the mounted state and the detached state according to the present embodiment. FIG. 5C illustrates that the HMD 30 is changed from the detached state to the mounted state in timing S1, illustrates that the HMD 30 is in the mounted state in a period from timing S1 to timing S2, illustrates that the HMD 30 is changed from the mounted state to the detached state in timing S2, illustrates that the HMD 30 is in the detached state in a period from timing S2 to timing S3, illustrates that the HMD 30 is changed from the detached state to the mounted state in timing S3, and illustrates that the HMD 30 is in the mounted state after timing S3. As illustrated in FIG. 5C, first, addition to the cumulative wearing time is performed in a period in which it is determined that the HMD 30 is in the mounted state (timing S1 to timing S2). Next, addition to the cumulative detachment time is performed in a period in which it is determined that the HMD 30 is in the detached state (timing S2 to timing S3), and no addition to the cumulative wearing time is performed in this period. Then, the cumulative wearing time is reset in timing Sd in which the cumulative detachment time exceeds a predetermined threshold value (Td). Then, addition to the cumulative wearing time is performed again from the reset state after timing S3 in which the HMD 30 is changed from the detached state to the mounted state. In addition, suppose that the cumulative detachment time is reset in timing S3 in which the HMD 30 is changed from the detached state to the mounted state. Incidentally, suppose that in FIG. 5C, the cumulative wearing time is reset also in timing Sa in which the cumulative wearing time exceeds a predetermined threshold value (Ta).

Incidentally, the cumulative detachment time may be reset in timing Sd in which the cumulative detachment time exceeds the predetermined threshold value (Td). In this case, the cumulative detachment time is not reset in timing in which the HMD 30 is changed from the detached state to the mounted state, and during the period during which it is determined that the HMD 30 is in the mounted state, the cumulative detachment time may be maintained at the value of the cumulative wearing time in timing in which the HMD 30 is changed from the detached state to the mounted state.

In addition, the counting unit 52 may perform subtraction from the cumulative wearing time in a period in which it is determined that the HMD 30 is in the detached state. In this case, because it is considered that the degree of fatigue of the user decreases according to the cumulative detachment time, or in other words, a break time during which the HMD 30 is not used, subtraction from the cumulative wearing time is performed according to the cumulative detachment time. That is, as the cumulative detachment time is lengthened, an amount of subtraction from the cumulative wearing time is increased, and therefore the degree of fatigue of the user tends to be evaluated as lower in user fatigue degree evaluation processing by the fatigue degree evaluating unit 53 to be described later.

FIG. 5D is a diagram illustrating an example of a cumulative wearing time graph corresponding to the mounted state and the detached state according to the present embodiment. FIG. 5D illustrates that the HMD 30 is changed from the detached state to the mounted state in timing S1, illustrates that the HMD 30 is in the mounted state in a period from timing S1 to timing S2, illustrates that the HMD 30 is changed from the mounted state to the detached state in timing S2, illustrates that the HMD 30 is in the detached state in a period from timing S2 to timing S3, illustrates that the HMD 30 is changed from the detached state to the mounted state in timing S3, and illustrates that the HMD 30 is in the mounted state after timing S3. As illustrated in FIG. 5D, first, addition to the cumulative wearing time is performed in a period in which it is determined that the HMD 30 is in the mounted state (timing S1 to timing S2). Next, in a period in which it is determined that the HMD 30 is in the detached state (timing S2 to timing S3), subtraction from the cumulative wearing time is performed. Then, addition to the cumulative wearing time is performed again after timing S3 after which it is determined that the HMD 30 is in the mounted state. Incidentally, suppose that in FIG. 5D, the cumulative wearing time is reset also in timing Sa in which the cumulative wearing time exceeds a predetermined threshold value (Ta).

The fatigue degree evaluating unit 53 evaluates the degree of fatigue of the user wearing the HMD 30 on the basis of the cumulative wearing time calculated by the counting unit 52. For example, with the cumulative wearing time as an index of the degree of fatigue of the user, the fatigue degree evaluating unit 53 in the present embodiment can perform evaluation such that the more an amount by which the cumulative wearing time is longer than the predetermined threshold value (Ta), the higher the degree of fatigue, and the more an amount by which the cumulative wearing time is shorter than the predetermined threshold value (Ta), the lower the degree of fatigue. In addition, suppose that degrees of fatigue corresponding to the cumulative wearing time are set. For example, the degree of fatigue may be evaluated as low when the cumulative wearing time exceeds a first time T1, the degree of fatigue may be evaluated as medium when the cumulative wearing time exceeds a second time T2 (> time T1) longer than the first time T1, and the degree of fatigue may be evaluated as high when the cumulative wearing time exceeds a third time T3 (> time T2) longer than the second time T2. Then, suppose that the fatigue degree evaluating unit 53 in the present embodiment monitors, at a predetermined frame rate, whether or not the cumulative wearing time calculated by the counting unit 52 exceeds the predetermined threshold value (Ta) as described above.

The notifying unit 54 provides the user with a notification corresponding to the degree of fatigue of the user which degree is evaluated by the fatigue degree evaluating unit 53. Specifically, suppose that the notifying unit 54 outputs a predetermined notification to the HMD 30 when the fatigue degree evaluating unit 53 determines that the cumulative wearing time exceeds the predetermined threshold value (Ta). Then, suppose that the cumulative wearing time is reset after the notifying unit 54 outputs the predetermined notification to the HMD 30.

Here, it suffices for the notification made by the notifying unit 54 to be such a notification as to make the user aware of the fatigue of the user himself/herself. For example, a voice or an image "usage time has exceeded Ta (for example, two hours)" may be output to the HMD 30, or a warning lamp provided to the HMD 30 may be lit.

In addition, the notifying unit 54 may output stepwise notifications to the HMD 30 according to the cumulative wearing time. For example, a first notification may be output to the HMD 30 when the cumulative wearing time exceeds the first time T1, that is, when the fatigue degree evaluating unit 53 evaluates the degree of fatigue as low, a second notification may be output to the HMD 30 when the cumulative wearing time exceeds the second time T2, that is, when the fatigue degree evaluating unit 53 evaluates the degree of fatigue as medium, and a third notification may be output to the HMD 30 when the cumulative wearing time exceeds the third time T3, that is, when the fatigue degree evaluating unit 53 evaluates the degree of fatigue as high. In this case, notifications may be made stepwise by different means, by, for example, outputting a sound as the first notification to the HMD 30, outputting an image as the second notification to the HMD 30, and limiting operation in the game played by the user as the third notification.

First Cumulative Wearing Time Calculation Processing An example of cumulative wearing time calculation processing performed by the relay apparatus 20 according to the present embodiment will be described in the following with reference to a flowchart of FIG. 6. The present processing example is an example of processing corresponding to the cumulative wearing time graph of FIG. 5B. In this case, suppose that the relay apparatus 20 repeatedly performs the cumulative wearing time calculation processing at a predetermined frame rate (for example, at intervals of 1/60 of a second) during the power-on period of the HMD 30.

First, the state determining unit 51 monitors the state of the HMD 30 (S101). In this case, suppose that the state determining unit 51 determines whether or not the state of the HMD 30 is changed, at a predetermined frame rate (hereinafter referred to as a state monitoring cycle time). That is, the state determining unit 51 determines whether the HMD 30 is changed from the detached state to the mounted state, the HMD 30 is changed from the mounted state to the detached state, or the state of the HMD 30 is not changed.

Then, when the state determining unit 51 determines in processing S101 that the state of the HMD 30 is not changed, the state determining unit 51 identifies the value of the state flag (S103). Then, when the value of the state flag is identified as "1," the counting unit 52 adds a predetermined time to the cumulative wearing time (S104). Here, the counting unit 52 adds the state monitoring cycle time in processing S101. That is, the counting unit 52 adds an elapsed time from a time point that the state determining unit 51 performs determination processing in previous processing S101 to a time point that the state determining unit 51 performs determination processing in the present processing S101. Incidentally, the predetermined time added to the cumulative wearing time is not limited to the state monitoring cycle time, but a time set as appropriate may be added. A return is thereafter made to processing S101 to repeat the processing from S101 on down.

In addition, when the value of the state flag is identified as "0" in processing S103, a return is made to processing S101 to repeat the processing from S101 on down.

In addition, when the state determining unit 51 determines in processing S101 that the HMD 30 is changed from the detached state to the mounted state, the state determining unit 51 updates the value of the state flag to "1" (S102). A return is thereafter made to processing S101 to perform the processing from S101 on down.

In addition, when the state determining unit 51 determines in processing S101 that the HMD 30 is changed from the mounted state to the detached state, the state determining unit 51 updates the value of the state flag to "0" (S105).

The counting unit 52 then determines whether or not to reset the cumulative wearing time (S106). Here, suppose that the counting unit 52 determines whether or not to reset the cumulative wearing time according to the value of the state flag, for example. The counting unit 52 determines that the cumulative wearing time is to be reset when the value of the state flag is "0," and determines that the cumulative wearing time is not to be reset when the value of the state flag is "1."

When determining in processing S105 that the cumulative wearing time is to be reset (S106: Y), the counting unit 52 resets the cumulative wearing time (S107). In addition, when it is determined in processing S106 that the cumulative wearing time is not to be reset (S106: N), a return is made to processing S101 to repeat the processing from S101 on down.

As described above, in the present processing example, the processing from processing S101 to processing S107 is repeated at the predetermined frame rate.

Incidentally, processing S106 and processing S107 may be omitted in the cumulative wearing time calculation processing illustrated in FIG. 6. Specifically, the cumulative wearing time may not be reset before a return is made to processing S101 after the state determining unit 51 updates the state flag to "0" in processing S105. That is, this case corresponds to the cumulative wearing time graph illustrated in FIG. 5A, and the cumulative wearing time is maintained without being reset during the period during which it is determined that the HMD 30 is in the detached state. Incidentally, whether or not to reset the cumulative wearing time may be configured to be settable before a return is made to processing S101 after the state determining unit 51 updates the state flag to "0" in processing S105.

Thus, in the present processing example, addition to the cumulative wearing time is performed in a period in which it is determined that the HMD 30 is in the mounted state, and no addition to the cumulative wearing time is performed in a period in which it is determined that the HMD 30 is in the detached state. Hence, even when the HMD 30 is mounted or detached in a state in which power to the HMD 30 is on, addition to the cumulative wearing time is performed only in periods in which it is determined that the HMD 30 is in the mounted state. Thus, times during which the HMD 30 is actually mounted can be calculated properly.

Second Cumulative Wearing Time Calculation Processing Another example of the cumulative wearing time calculation processing performed by the relay apparatus 20 according to the present embodiment will next be described with reference to a flowchart of FIG. 7. The present processing example is an example of processing corresponding to the cumulative wearing time graph of FIG. 5C. In this case, suppose that the relay apparatus 20 repeatedly performs the cumulative wearing time calculation processing at a predetermined frame rate (for example, at intervals of 1/60 of a second) during the power-on period of the HMD 30. In addition, the processing of processing S201 to processing S205 in FIG. 7 is similar to processing of processing S101 to processing S105 in FIG. 6, and therefore repeated description thereof will be omitted in the following.

When it is determined in processing S203 that the value of the state flag is "0," the counting unit 52 adds a predetermined time to the cumulative detachment time (S206). Here, the counting unit 52 may add a state monitoring cycle time in processing S201, or may add a time set as appropriate.

The counting unit 52 then determines whether or not to reset the cumulative wearing time (S207). Here, suppose that the counting unit 52 determines whether or not to reset the cumulative wearing time according to the cumulative detachment time. In this case, the counting unit 52 determines that the cumulative wearing time is to be reset when the cumulative detachment time exceeds the predetermined threshold value (Td), and determines that the cumulative wearing time is not to be reset when the cumulative detachment time is equal to or less than the predetermined threshold value (Td).

When determining in processing S207 that the cumulative wearing time is to be reset (S207: Y), the counting unit 52 resets the cumulative wearing time (S208). A return is thereafter made to processing S201 to repeat the processing from S201 on down. In addition, when it is determined in processing S207 that the cumulative wearing time is not to be reset (S207: N), a return is made to processing S201 to repeat the processing from S201 on down.

In addition, when the value of the state flag is updated to "1" in processing S202, the counting unit 52 resets the cumulative detachment time (S209). A return is thereafter made to processing S201 to repeat the processing from S201 on down.

In addition, after the state flag is updated to "0" in processing S205, a return is made to processing S201 to repeat the processing from S201 on down.

As described above, in the present processing example, the processing from processing S201 to processing S209 is repeated at the predetermined frame rate.

Incidentally, the processing of processing S209 may be performed in processing S208 in the cumulative wearing time calculation processing illustrated in FIG. 7. Specifically, when determining that the cumulative wearing time is to be reset in processing S207 (S207: Y), the counting unit 52 may reset the cumulative wearing time and the cumulative detachment time in processing S208. In this case, the cumulative detachment time is not reset when the state flag is updated to 1 in processing S202. Thus, the cumulative detachment time is maintained in a period in which it is determined that the HMD 30 is in the mounted state, and the predetermined time is added to the maintained cumulative detachment time in a next period in which it is determined that the HMD 30 is in the detached state.

Thus, in the present processing example, addition to the cumulative wearing time is performed in a period in which it is determined that the HMD 30 is in the mounted state, and no addition to the cumulative wearing time is performed and addition to the cumulative detachment time is performed in a period in which it is determined that the HMD 30 is in the detached state. Then, the cumulative wearing time is reset according to the calculated cumulative detachment time. Hence, even when the HMD 30 is mounted or detached in a state in which power to the HMD 30 is on, times during which the HMD 30 is actually mounted can be calculated properly, and the cumulative wearing time can be controlled according to the cumulative detachment time.

In addition, the counting unit 52 may further subtract the predetermined time from the cumulative wearing time in processing S206 of the cumulative wearing time calculation processing illustrated in FIG. 7. This case corresponds to the cumulative wearing time graph illustrated in FIG. 5D. Here, the counting unit 52 may subtract the state monitoring cycle time (Tc) from the cumulative wearing time, may subtract a·Tc (a is a coefficient less than one) from the cumulative wearing time, or may subtract another time set as appropriate. It is thereby possible to calculate the cumulative wearing time in consideration of the cumulative detachment time.

Incidentally, after the counting unit 52 performs addition to the cumulative wearing time in processing S104 of FIG. 6 or processing S204 of FIG. 7 described above, the fatigue degree evaluating unit 53 may determine whether or not the cumulative wearing time to which addition has been performed exceeds the predetermined threshold value (Ta). Then, when the fatigue degree evaluating unit 53 determines that the cumulative wearing time to which addition has been performed exceeds the predetermined threshold value (Ta), the notifying unit 54 may output a predetermined notification to the HMD 30.

### First Modification

In addition, while the above-described example assumes a case in which the HMD 30 is used by one user, the present invention is also applicable to a case in which a plurality of users use one HMD 30. Suppose that in a case in which a plurality of users use one HMD 30, the users wearing the HMD 30 are identified, and the cumulative wearing time is calculated for each of the identified users. Then, suppose that when the cumulative wearing time of an identified user exceeds a predetermined threshold value, a notification is provided to the user.

Specifically, suppose that the sensor unit of the HMD 30 includes an imaging element such as a CCD image sensor, a complementary metal oxide semiconductor (CMOS) image sensor, an infrared image sensor, or the like, and images a face image of a user when the HMD 30 is mounted on the user. Then, suppose that the state determining unit 51 identifies the user wearing the HMD 30 from feature information extracted from the imaged face image of the user by using a face recognition technology or an iris recognition technology that is publicly known. In addition, the identification of the user wearing the HMD 30 is not limited to the above example. For example, the user may be identified by using feature information extracted by publicly known voice recognition from a voice uttered by the user, the sound of the voice being collected by a microphone unit provided to the HMD 30, or the user may be identified by making the user wearing the HMD 30 perform login operation. In addition, the user may be identified by using an image of the user imaged by an external camera unit. In this case, suppose, for example, that user information associating feature information with personal information (a user name, a password, a name, an age, or the like) of the user is registered in the entertainment apparatus 10 in advance. The state determining unit 51 can identify the user name or the like of the user wearing the HMD 30 by referring to the registered user information. Incidentally, the user information may be registered in the relay apparatus 20, the HMD 30, or another server.

Then, suppose that the state determining unit 51 retains wearing user information together with the state flag. In this case, suppose that information indicating the user wearing the HMD 30 in a period in which it is determined that the HMD 30 is in the mounted state is stored as the wearing user information, and the user name or the like obtained from the user information described above may be stored as the wearing user information. Specifically, suppose that when the state determining unit 51 determines that the HMD 30 is changed from the detached state to the mounted state in processing S101 of FIG. 6 or processing S201 of FIG. 7, the state determining unit 51 identifies the user wearing the HMD 30 by a method as described above. Then, in processing S102 of FIG. 6 or processing S202 of FIG. 7, the state determining unit 51 updates the value of the state flag to "1," and updates the wearing user information to information (for example, the user name) indicating the identified user.

Then, suppose that for each user identified by the state determining unit 51, the counting unit 52 retains cumulative wearing time data in association with the information indicating the user. Specifically, suppose that when the value of the state flag is identified as "1" in processing S103 of FIG. 6 or processing S203 of FIG. 7, the state determining unit 51 identifies the information indicating the user which information is retained as the wearing user information. Then, suppose that in processing S104 of FIG. 6 or processing S204 of FIG. 7, the counting unit 52 performs addition to the cumulative wearing time using the cumulative wearing time data associated with the information indicating the user which information is identified by the state determining unit 51.

Incidentally, while the state determining unit 51 identifies the user wearing the HMD 30 in the above-described example, it suffices to be able only to distinguish the user wearing the HMD 30 in calculating the cumulative wearing time for each user. That is, even when the user wearing the HMD 30 is not identified, it suffices to be able to distinguish a plurality of users wearing the HMD 30. For example, the state determining unit 51 may distinguish the plurality of users by adding identifiers such as a first user, a second user, and a third user to feature information extracted from face images of the users imaged by the sensor unit 36 of the HMD 30. In addition, the state determining unit 51 may extract feature information (for example, the colors of clothes, physical constitutions, or the like) that can distinguish the plurality of users from images of the users imaged by the external camera unit, and add the identifiers. In this case, suppose that the identifiers are stored as the wearing user information retained by the state determining unit 51, and that the counting unit 52 retains the cumulative wearing time data in association with the identifiers.

Thus, even in a case where a plurality of users alternately wear the HMD 30, a proper cumulative wearing time can be calculated for each user, that is, a degree of user fatigue can be evaluated properly for each user. Then, a notification can be provided to each user in appropriate timing.

### Second Modification

The foregoing embodiment evaluates the degree of fatigue of a user using the HMD 30 on the basis of the cumulative wearing time, from an idea that the degree of fatigue of the user using the HMD 30 increases according to an increase in the cumulative wearing time. Here, there is no limitation to the example in which the degree of fatigue of the user increases according to an increase in the cumulative wearing time, but there may be cases where the degree of fatigue of the user differs according to display contents of the HMD 30, the attributes of the user using the HMD 30, and the like. For example, even when a time of wearing the HMD 30 is the same in a case where a three-dimensional video is viewed using the HMD 30 and in a case where a two-dimensional video is viewed using the HMD 30, it is considered that the degree of fatigue of the user is higher in the case where the three-dimensional video is viewed. Thus, it may not be possible to evaluate the degree of fatigue of the user properly by only simply evaluating the degree of fatigue of the user on the basis of the cumulative wearing time obtained by accumulating times in which it is determined that the HMD 30 is in the mounted state.

Accordingly, the degree of fatigue of the user may be evaluated on the basis of information about the user wearing the HMD 30, video displayed by the HMD 30, or the like in a period in which it is determined that the HMD 30 is in the mounted state. Specifically, when the counting unit 52 adds the predetermined time to the cumulative wearing time, the predetermined time to be added may be controlled according to various kinds of conditions (the information about the user, the video displayed by the HMD 30, and the like).

For example, the counting unit 52 may obtain the user information about the user identified by the state determining unit 51 as described above, and control the predetermined time to be added according to the user information. For example, when the age of the identified user satisfies a predetermined condition (for example, 15 years old or under or 65 years old or over), the counting unit 52 may add b·Tc (b is a coefficient larger than one) to the cumulative wearing time. Consequently, a young or elderly user who easily accumulates fatigue through the use of the HMD 30 is readily provided with a notification corresponding to the degree of fatigue of the user. Incidentally, when the age of the identified user does not satisfy the predetermined condition (for example, 16 years old or over and 64 years old or under), the counting unit 52 may add d·Tc (d is a coefficient smaller than one) to the cumulative wearing time. Consequently, a user of an average age who uses the HMD 30 is provided with a notification corresponding to the degree of fatigue of the user less readily than a young or elderly user.

In addition, information about an application used by the user in a period in which the HMD 30 is mounted may be obtained, and the predetermined time added by the counting unit 52 may be controlled according to the application used. For example, information about an application executed by the entertainment apparatus 10 may be obtained from the entertainment apparatus 10, and when it is determined that the application displays three-dimensional video, the counting unit 52 may add b·Tc (b is a coefficient larger than one) to the cumulative wearing time. In addition, information about video displayed on the display unit 34 of the HMD 30 may be obtained from the HMD 30, and when it is determined that three-dimensional video is displayed, the counting unit 52 may add b·Tc (b is a coefficient larger than one) to the cumulative wearing time. Consequently, a user viewing three-dimensional video that relatively easily causes fatigue to be accumulated is readily provided with a notification corresponding to the degree of fatigue of the user. Incidentally, when it is determined that the application being used by the user is not an application displaying three-dimensional video, the counting unit 52 may add d·Tc (d is a coefficient smaller than one) to the cumulative wearing time. Consequently, a user viewing two-dimensional video or the like that does not relatively easily cause fatigue to be accumulated is provided with a notification corresponding to the degree of fatigue of the user less readily than a user viewing three-dimensional video.

In addition, information about an application executed by the entertainment apparatus 10 may be obtained from the entertainment apparatus 10, and when it is determined that the application includes video with violent movement, video blinking frequently, or video that relatively easily causes fatigue to be accumulated, the counting unit 52 may add b·Tc (b is a coefficient larger than one) to the cumulative wearing time. The application including video that relatively easily causes fatigue to be accumulated may, for example, be a game application, or may be the application of a battle game, the application of a race game, or the like, which is considered to cause fatigue to be accumulated particularly easily among game applications. Incidentally, when the application being executed is not a game application, the counting unit 52 may add d·Tc (d is a coefficient smaller than one) to the cumulative wearing time.

In addition, the counting unit 52 may add b·Tc (b is a coefficient larger than one) to the cumulative wearing time in a period in which the display unit 34 of the HMD 30 displays video that easily causes fatigue to be accumulated as described above (three-dimensional video, video with violent movement, blinking video, or the like) among periods in which the HMD 30 is mounted.

It is to be noted that the present invention is not limited to the foregoing embodiment.

For example, a part or all of functions implemented by the relay apparatus 20 according to the present embodiment which relay apparatus is illustrated in FIG. 4 may be implemented in the entertainment apparatus 10, or may be implemented in the HMD 30. Alternatively, functions illustrated in FIG. 4 may be implemented in a distributed manner in the entertainment apparatus 10, the relay apparatus 20, or the HMD 30.

## Claims

1. An information processing apparatus comprising:
a state determining unit configured to determine whether a head-mounted display is in a mounted state;
a fatigue degree evaluating unit configured to evaluate a degree of fatigue of a user wearing the head-mounted display on a basis of a cumulative wearing time obtained by accumulating times in which it is determined that the head-mounted display is in the mounted state; and
a notifying unit configured to provide the user with a notification corresponding to the degree of fatigue of the user.

2. The information processing apparatus according to claim 1,
wherein
the notifying unit provides the notification when the cumulative wearing time exceeds a predetermined threshold value.

3. The information processing apparatus according to claim 1 or 2,
wherein
the state determining unit is able to further determine whether the head-mounted display is in a detached state, and
the cumulative wearing time is reset when a cumulative detachment time obtained by accumulating times in which it is determined that the head-mounted display is in the detached state exceeds a predetermined threshold value.

4. The information processing apparatus according to claim 1 or 2,
wherein
the state determining unit is able to further determine
whether the head-mounted display is in a detached state, and subtraction from the cumulative wearing time is performed according to a time in which it is determined that the head-mounted display is in the detached state.

5. The information processing apparatus according to claim 1 or 2,
wherein
the state determining unit is able to further determine
whether the head-mounted display is in a detached state, and the cumulative wearing time is reset when it is determined that the head-mounted display is in the detached state.

6. The information processing apparatus according to claim 1, further comprising:
a user identifying unit configured to identify the user wearing the head-mounted display, wherein
the fatigue degree evaluating unit evaluates the degree of fatigue of the user on the basis of the cumulative wearing time obtained by accumulating times in which it is determined that the identified user wears the head-mounted display, and the notifying unit provides the notification to the user when the cumulative wearing time of the identified user exceeds a predetermined threshold value.

7. A fatigue degree evaluating method comprising:
a step of determining whether a head-mounted display is in a mounted state; and
a step of evaluating a degree of fatigue on a basis of a cumulative wearing time obtained by accumulating times in which it is determined that the head-mounted display is in the mounted state.

8. A program for making a computer function as:
state determining means for determining whether a head-mounted display is in a mounted state;
fatigue degree evaluating means for evaluating a degree of fatigue of a user wearing the head-mounted display on a basis of a cumulative wearing time obtained by accumulating times in which it is determined that the head-mounted display is in the mounted state; and
notifying means for providing the user with a notification corresponding to the degree of fatigue of the user.

## Amended claims

### Amended claims under Art. 19.1 PCT

**1.** (Amended) An information processing apparatus comprising:
a state determining unit configured to determine whether a head-mounted display is in a mounted state;
a fatigue degree evaluating unit configured to evaluate a degree of fatigue of a user wearing the head-mounted display on a basis of a cumulative wearing time obtained by accumulating times in which it is determined that the head-mounted display is in the mounted state; and
a notifying unit configured to provide the user with a notification corresponding to the degree of fatigue of the user;
the state determining unit being able to further determine whether the head-mounted display is in a detached state;
the cumulative wearing time being reset when a cumulative detachment time obtained by accumulating times in which it is determined that the head-mounted display is in the detached state exceeds a predetermined threshold value.

**2.** The information processing apparatus according to claim 1,
wherein
the notifying unit provides the notification when the cumulative wearing time exceeds a predetermined threshold value.

**3.** (Amended) The information processing apparatus according to claim 1 or 2, wherein
subtraction from the cumulative wearing time is performed according to a time in which it is determined that the head-mounted display is in the detached state.

**4.** (Amended) The information processing apparatus according to claim 1, further comprising:
a user identifying unit configured to identify the user wearing the head-mounted display,
wherein
the fatigue degree evaluating unit evaluates the degree of fatigue of the user on the basis of the cumulative wearing time obtained by accumulating times in which it is determined that the identified user wears the head-mounted display, and the notifying unit provides the notification to the user when the cumulative wearing time of the identified user exceeds a predetermined threshold value.

**5.** (Amended) A fatigue degree evaluating method comprising:
a step of determining whether a head-mounted display is in a mounted state;
a step of evaluating a degree of fatigue on a basis of a cumulative wearing time obtained by accumulating times in which it is determined that the head-mounted display is in the mounted state;
a step of determining whether the head-mounted display is in a detached state; and
a step of resetting the cumulative wearing time when a cumulative detachment time obtained by accumulating times in which it is determined that the head-mounted display is in the detached state exceeds a predetermined threshold value.

**6.** (Amended) A program for making a computer function as: state determining means for determining whether a head-mounted display is in a mounted state;
fatigue degree evaluating means for evaluating a degree of fatigue of a user wearing the head-mounted display on a basis of a cumulative wearing time obtained by accumulating times in which it is determined that the head-mounted display is in the mounted state; and
notifying means for providing the user with a notification corresponding to the degree of fatigue of the user;
the state determining unit being able to further determine whether the head-mounted display is in a detached state;
the cumulative wearing time being reset when a cumulative detachment time obtained by accumulating times in which it is determined that the head-mounted display is in the detached state exceeds a predetermined threshold value.

Statement under Art. 19.1 PCT
A configuration according to claim 3 before amendment is added to claims 1, 5, and 6, in which configuration "the state determining unit can further determine whether the head-mounted display is in a detached state, and the cumulative wearing time is reset when a cumulative detachment time obtained by accumulating times in which it is determined that the head-mounted display is in the detached state exceeds a predetermined threshold value." Document 2 cited in the international search report describes resetting a usage duration when the absence time of a user exceeds a certain time, and resetting the absence time when the user returns before the absence time exceeds the certain time. It is obvious from this description that absence times are not accumulated in Document 2, and the above-described configuration cannot be said to be disclosed nor suggested in Document 2.

In addition, the above-described configuration is not disclosed nor suggested in Documents 1, 3, and 4 cited in the international search report.
